# EUROPEAN PATENT SPECIFICATION

(11) **EP 3 216 447 B1**
(45) Date of publication and mention of the grant of the patent: **02.06.2021**
(21) Application number: 15857765.0
(22) Date of filing: 05.11.2015
(51) Int. Cl.: A61K 31/4152, A61K 9/08, A61K 9/20, A61K 9/48, A61P 25/00, A61K 45/06, A61P 25/28, A61K 39/395

(54) **USE OF EDARAVONE IN PREPARING DRUG FOR PREVENTING AND TREATING CEREBRAL AMYLOID ANGIOPATHY (CAA)**
VERWENDUNG VON EDARAVON ZUR HERSTELLUNG EINES ARZNEIMITTELS ZUR PRÄVENTION UND BEHANDLUNG VON AMYLOIDER GEHIRNANGIOPATHIE (CAA)
UTILISATION D'ÉDARAVONE DANS LA PRÉPARATION DE MÉDICAMENT POUR LA PRÉVENTION ET LE TRAITEMENT DE L'ANGIOPATHIE AMYLOÏDE CÉRÉBRALE (AAC)

(30) Priority: 05.11.2014 CN 201410618300
(43) Date of publication of application: 13.09.2017
(73) Proprietor: The Third Affiliated Hospital Third Military Medical University, PLA, Yuzhong District, Chongqing 400042 (CN)
(72) Inventor: WANG, Yanjiang, Chongqing 400042 (CN); JIAO, Shusheng, Chongqing 400042 (CN); YAO, Xiuqing, Chongqing 400042 (CN); ZHOU, Xinfu, Adelaide, SA 5000 (AU)
(74) Representative: Dörr, Klaus
(86) International application number: PCT/CN2015/093837
(87) International publication number: WO 2016/070817

(56) References cited:
- WO-A1-2013/138368
- WO-A1-2014/089500
- WO-A2-03/015691
- DATABASE CA [Online] CHEMICAL ABSTRACTS SERVICE, COLUMBUS, OHIO, US; 2 June 2011 (2011-06-02), LI, MINGQIU ET AL: "Clinical observation for slight and moderate Alzheimer treated with edaravone", XP002781039, retrieved from STN Database accession no. 2011:685086
- FENG HE ET AL: "Inhibitory Effects of Edaravone in [beta] -Amyloid-Induced Neurotoxicity in Rats", BIOMED RESEARCH INTERNATIONAL, vol. 2014, 1 January 2014 (2014-01-01), pages 1-7, XP055474905, ISSN: 2314-6133, DOI: 10.1155/2014/370368
- DATABASE CA [Online] CHEMICAL ABSTRACTS SERVICE, COLUMBUS, OHIO, US; 2010, LI, LEI ET AL: "Effects of edaravone on learning and memory ability and the Mrna expression of .beta.- amyloid precursor protein in the hippocampus of Alzheimer's disease model rats", XP002781040, retrieved from STN Database accession no. 2010:1448794
- ZHOU SHANSHAN ET AL: "Neuroprotective effects of edaravone on cognitive deficit, oxidative stress and tau hyperphosphorylation induced by intracerebroventricular streptozotocin in rats", NEUROTOXICOLOGY, vol. 38, 7 August 2013 (2013-08-07), pages 136-145, XP028711077, ISSN: 0161-813X, DOI: 10.1016/J.NEURO.2013.07.007
- T. NAKAMURA ET AL: "Edaravone Attenuates Brain Edema and Neurologic Deficits in a Rat Model of Acute Intracerebral Hemorrhage", STROKE, vol. 39, no. 2, 1 February 2008 (2008-02-01), pages 463-469, XP055475114, US ISSN: 0039-2499, DOI: 10.1161/STROKEAHA.107.486654
- SHEN YUE-E. ET AL.: 'Effects of edaravone on amyloid-B precursor protein processing in sy5y-app695 cells' NEUROTOXICITY RESEARCH vol. 24, no. 2, 17 January 2013, ISSN 1029-8428 pages 139 - 147, XP055278284
- WANG, JIANHUA ET AL.: 'Effects of Edaravone on the expressions of Abeta Tau and GFAP to the brain of vascular dementia in rat' CHINESE JOURNAL OF NEUROANATOMY vol. 27, no. 2, 31 March 2011, page 204, XP009502830

## Description

### FIELD OF THE INVENTION

The present invention belongs to the pharmaceutical field. Specifically, the present application relates to the use of Edaravone for preventing and scavenging intracerebral Aβ deposition, in particular for preventing and treating cerebral amyloid angiopathy (CAA).

### BACKGROUND

Cerebral amyloid angiopathy (CAA) refers to the deposition of β-amyloid (Aβ) on the media and adventitia of small and mid-sized arteries (and, less frequently, veins) of the cerebral cortex and the medulla. It is a component of any disorder in which amyloid is deposited in the brain. CAA has been recognized as one of the morphologic hallmarks of Alzheimer disease (AD), but it is also often found in the brains of elderly patients who are neurologically healthy. While often asymptomatic, CAA may lead to intracranial hemorrhage (ICH), dementia or transient neurologic events, in which ICH is the most recognized result. Effective therapeutics for CAA is currently not available.

Edaravone, chemical structure being 3-methyl-1-phenyl-2-pyrazolin-5-one and molecular formula being C₁₀H₁₀N₂O, contains three antioxidant groups (see, for example, Edaravone (3-methyl-1-phenyl-2-pyrazolin-5-one), a novel free radical scavenger, for treatment of cardiovascular diseases, Higashi Y1, Jitsuiki D, Chayama K, Yoshizumi M., Recent Pat Cardiovasc Drug Discov., Jan. 2006; 1(1):85-93, and The reaction rate of edaravone (3-methyl-1-phenyl-2-pyrazolin-5-one (MCI-186)) with hydroxyl radical, Abe S, Kirima K, Tsuchiya K, Okamoto M, Hasegawa T , Houchi H, Yoshizumi M , Tamaki T., Chem Pharm Bull (Tokyo), Feb. 2004; 52(2): 186-91), which has potent antioxidant effects and radical scavenging effects. It is a medicine currently approved for treating acute ischemic stroke in clinical practice, which exerts neuroprotective effects by scavenging the oxygen free radicals generated during the ischemia-reperfusion. It is found in the previous studies that Edaravone could reduce cerebral ischemia-reperfusion injury area, and suppress the gene expression of the Fas/FasL signaling pathway and thereby inhibit neuronal apoptosis (see, for example, Edaravone neuroprotection effected by suppressing the gene expression of the Fas signal pathway following transient focal ischemia in rats, Xiao B, Bi FF, Hu YQ, Tian FF, Wu ZG, Mujlli HM, Ding L, Zhou XF, Neurotox Res., Oct. 2007; 12(3):155-62). It is found in a recent research that Edaravone could inhibit the mitochondrion dependant apoptosis pathway in the N2a/ Swe.Δ9 cells. In the ischemia-reperfused elderly rats, Edaravone could reduce the glutathione peroxidase activity, suppress the JNK-c-Jun pathway and decrease the neuronal apoptosis. Edaravone has therapeutic effects on other nervous system degenerative diseases such as amyotrophic lateral sclerosis (ALS) and Parkinson's disease. Li, Mingqui et al. (2011, Chemical Abstract Service, acc. No: 2011:685086) discloses the use of Edaravone together with donezepil (Alzheimer's (AD) treatment medicament) by injection of 30 mg/day in AD therapy. Feng He et al. (2014, BioMed Research International, vol. 2014, 1-7) teaches Edaravone as an antioxidative agent in the treatment of AD in rats, reports that Edaravone has been shown to produce neuroprotective effects and discloses an effect of Edaravone in the treatment of AD. Li, Lei et al. (2010, Chemical Abstract Service, acc. No: 2010:1448794) teaches administering Edaravone by intravenous injection to lower the expression of beta-amyloid precursor protein (APP) in the context of AD in rats. Shanshan Zhou et al. (2013, NeuroToxicology, vol. 38, 136-145) investigated the effects of Edaravone on oxidative stress and neuronal degeneration in the context of AD. T. Nakamura et al. (2008, Stroke, vol 39, no. 2, 463-469) teaches that Edaravone attenuates brain edema and neurological deficits in a rat model of acute intracerebral hemorrhage. WO 2014/089500 A1 discloses the use of an anti-Aβ antibody or antigen-binding fragment thereof to reduce brain amyloid plaques, or minimize the occurrence of microhemorrhage during chronic dosing of an anti-Aβ antibody or antigen-binding fragment thereof. WO 03/015691 A2 discloses a method for improvement in cognition in subjects suffering from conditions or diseases related to the Aβ peptide, including Alzheimer's disease, Down's syndrome, cerebral amyloid angiopathy, mild cognitive impairment, and the like. The method comprises administering anti-Aβ antibodies to the subject, especially antibodies having a high affinity for soluble forms of Aβ. WO 2013/138368 A1 discloses a substance for treating a cerebral amyloid angiopathy related condition or disease affecting cerebrovasculature in a patient, comprising an inhibitor that causes inhibition of the formation of membrane attack complex of the complement system and a vehicle for transporting the inhibitor into the cerebrovasculature; where the inhibition by the inhibitor is sufficient to decrease the incidence of or to prevent the incidence of cytolysis of the smooth muscle cells.

### SUMMARY OF THE INVENTION

It is firstly discovered by the present inventors that Edaravone directly acts on Aβ, inhibits Aβ aggregation, promotes Aβ fiber disaggregation, influences Aβ metabolism, thereby blocks or delays the progression of cerebral amyloid angiopathy (CAA).

The present invention provides Edaravone, an Edaravone analogue, wherein (1) the methyl at position 3 of the pyrazoline ring is replaced with a lower (CI-6) alkyl other than methyl, or with a lower alkoxy, (2) the methyl at position 3 of the pyrazoline ring is replaced with hydrogen while the hydrogen at position 4 is substituted with a lower alkyl or alkoxy, and/or (3) phenyl is optionally substituted with one or more substituents selected from a lower alkyl, a lower alkoxy, nitro, and halogen, or an Edaravone derivative, wherein the ketone in position 5 of the pyrazoline ring is transformed into enol that can react with a carboxylic acid to generate an ester, which in turn can be converted into a ketone after hydrolysis in vivo, for use in the treatment of cerebral amyloid angiopathy (CAA) in a patient.

According to the present invention, Edaravone is administered intravenously, subcutaneously or orally, preferably administered orally.

Edaravone can be formulated into an injection, a tablet, a capsule, etc.

Edaravone can be administered in combination with other CAA therapeutic medicine, such as an Aβ antibody, if desired.

According to the present invention, the method further comprises determining that the patient suffers from CAA, wherein the determination step occurs before the administration step. The determination step is for determining the clinical symptoms of the patient suffering from CAA.

According to the present invention, the patient has or does not have characteristic plaques of Alzheimer's disease in the brain.

According to the present disclosure, the patient has or does not have symptoms of Alzheimer's disease.

According to the present disclosure, the patient has experienced or has not experienced a heart attack or stroke.

According to the present invention, the daily dose of Edaravone is between about 0.1 mg/kg and about 25 mg/kg. The daily dose is preferably 0.5-5.0 mg/kg for prevention purpose, while preferably 5.0-25mg/kg for treatment purpose. For example, the daily dose is about 0.5 mg/kg, 3.5 mg/kg or 15 mg/kg, bid or biw.

According to the present disclosure, the method further comprises monitoring the change of the signs or symptoms corresponding to the CAA during administration period.

The present invention further provides Edaravone, an Edaravone analogue wherein (1) the methyl at position 3 of the pyrazoline ring is replaced with a lower (C1-6) alkyl other than methyl, or with a lower alkoxy, (2) the methyl at position 3 of the pyrazoline ring is replaced with hydrogen while the hydrogen at position 4 is substituted with a lower alkyl or alkoxy, and/or (3) phenyl is optionally substituted with one or more substituents selected from a lower alkyl, a lower alkoxy, nitro, and halogen, or an Edaravone derivative wherein the ketone in position 5 of the pyrazoline ring is transformed into enol that can react with a carboxylic acid to generate an ester, which in turn can be converted into a ketone after hydrolysis in vivo, for use in the prevention of CAA in a patient susceptible to CAA.

The present disclosure further provides a method for reducing a vascular amyloid protein of a patient, comprising administering Edaravone according to a therapeutic regime that is related to the removal of the vascular amyloid protein and reduces the incidence of cerebral microbleeds. The method further comprises monitoring the cerebral microbleeds of the patient by MRI. The method further comprises monitoring the removal of the vascular amyloid protein of the patient by a PET scan. The therapeutic regime can be a long-term therapeutic regime.

### Edaravone and analogues or derivatives thereof

The Edaravone has the chemical name of 3-methyl-1-pheneyl-2-pyrazolin-5-one, molecular formula of C₁₀H₁₀N₂O, molecular weight of 174.19, and the chemical structural formula of

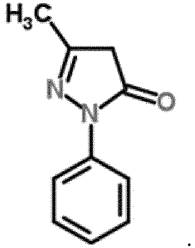

Analogues of Edaravone comprise those in which methyl at the position 3 of the pyrazoline ring may be replaced with a lower (C₁₋₆) alkyl such as ethyl, propyl, etc., or replaced with a lower alkoxy such as methoxy, ethoxy, etc., or methyl at position 3 may be replaced with H while the position 4 is substituted with a lower alkyl or alkoxy. Derivatives of Edaravone comprise esters, i.e. those in which ketone in position 5 of the pyrazoline ring is transformed into enol, and reacts with a carboxylic acid to generate esters such as methyl esters, ethyl esters, etc. The ester (precursor) is converted again into ketone after hydrolysis in vivo. In addition, phenyl is also optionally substituted with one or more substitients that are selected from a lower alkyl, a lower alkoxy, nitro, halogen, etc.

It is expected in the present invention that the above analogues and derivatives have the equivalent function with Edaravone.

It is reported that a hereditary factor plays a role in certain types of CAA and CAA-related diseases. Therefore, it is optionally to determine the symptom, sign or the presence or absence of the risk factors of the disease before treatment.

### Diagnosis and the monitoring of a CAA patient

Like most neurologic diseases, diagnosis often comes up on the basis of a medical history of the patient, a careful inquiry of the family history, onset cases of the patient, a symptomatic pattern, as well as a neurological examination. Brain computerized tomoscanning (CT) or magnetic resonance imaging (MRI) may identify lobar hemorrhage, stroke or petechial hemorrhage, which are very important in excluding arteriovenous malformation, brain tumor or other hemorrhage causes. Angiography (an X-ray test of the inside of blood vessel and heart) is no help for the diagnosis of CAA, but it may be desired for excluding aneurysm. Brain biopsy (excision of small pieces of brain tissue) could show a characteristic amyloid deposit. When the diagnosis is uncertain, biopsy may be needed to exclude potential diseases to be treated. Lumbar puncture that determines a cerebrospinal fluid protein could show a characteristic abnormality.

CAA accompanied by hemorrhage must be distinguished from the other types of cerebral hemorrhage. In CAA, hemorrhage usually occurs in the lobe, the blood, after rupture, often enters the subarachnoid space between brain and the capsule during the night. In hypertension correlated hemorrhage, hemorrhage often occurs deeper in the brain, the blood, after rupture, enters the chamber or cavity deep in the brain during the daytime activities. Other causes for brain hemorrhage include arteriovenous malformation, wound, aneurysm, bleeding in brain tumors, vasculitis (vascular inflammation) or hemorrhagic disorders. Cerabral microbleeds of the patient may be monitored by MRI and/or the removal of the vascular amyloid protein of the patient may be monitored by a positron emission tomography (PET) scan.

Patients suitable for treatment include the individuals that are under the risk of CAA while no symptom is shown, as well as the patients who are currently showing the symptoms.

### Therapeutic regimes

In the prophylactic use, the regime for administering a pharmaceutical composition or agent to the patient susceptible to CAA or under the risk of CAA comprises administering the composition or agent with an amount and frequency sufficient to eliminate or reduce the risk, mitigate the severity or delay the onset of disease, including the physiological, biochemical, historical and/or behavioral symptoms of the disease, complications and intermediate pathological phenotypes shown during the development of the disease. In the therapeutic use, the regime for administering a pharmaceutical composition or agent to the patient having or being suspected of having such disease comprises administering the composition with an amount and frequency sufficient to cure or at least partially suppress the symptom of the disease, including the complications and intermediate pathological phenotypes shown during the development of the disease. The appropriate amount to complete the therapeutic or prophylactic therapy is defined as a therapeutically or prophylactically effective amount. The combination of the appropriate amount and administration frequency to complete therapeutic or prophylactic therapy is defined as a therapeutically or prophylactically effective regime.

The administration amount and frequency of the present agent may depend on the prophylactic or therapeutic application of the therapy. In the prophylactic application, a pharmaceutical composition comprising Edaravone is administered to the patient who is not yet at a state of disease to enhance his/her resistivity. This amount is defined as "a prophylactically effective amount". In this application, the precise amount still depends on the health status of the patient, but is usually at 0.1-25 mg per dose, especially in the range of 0.5-5.0 mg per dose. A relatively low amount is administered at a relatively infrequent interval over a long period of time. Some patients receive the permanent treatment in the rest of their lives.

In the therapeutic application, it may sometimes requires administering a relatively higher amount (for example, about 3.5-25 mg per dose, or 5.0-25 mg per dose) at a relatively shorter interval until the development of the disease mitigates or terminates, preferably until partial or complete improvement of the symptom is shown in the patient. After that, a prophylactic scheme may be applied to the patient.

The presently claimed pharmaceutical agent may optionally be administered in combination with other agents that are at least partially effective in the treatment of CAA or AD. In the case of CAA when an amyloid deposit is present in the cerebrovascular system, the presently claimed pharmaceutical agent may also be used in combination with other drugs that could enhance the migration of the presently claimed agent across the blood-brain barrier.

The presently claimed agent may be administered parenterally, topically, intravenously, orally, subcutaneously, intraarterially, intracranially, intrathecally, intraperitoneally, intranasally or intramuscularly for the prophylactic and/or therapeutic treatment. Although other routes can be equally effective, a typical route of administration is an oral administration, secondly an intramuscular injection. This kind of injection is most frequently carried out in the muscles of arms or legs. In some methods, the agent is directly injected into the specific tissues where the deposit accumulates, for example, by intracranial injection. Intramuscular injection or intravenous infusion is also preferred. In some methods, a specific therapeutic antibody is directly injected intracranially.

The beneficial effect of the present invention are mainly embodied in that Edaravone or the analogues or derivatives thereof have an effect of inhibiting Aβ aggregation, thereby Aβ that already deposited in the intracerebral vascular wall or in the cerabral parenchyma can be eliminated, or a continuous deposition of Aβ in the intracerebral vascular wall and the cerabral parenchyma can be prevented.

### BRIEF DESCRIPTION OF THE DRAWINGS

For more clearly describing the technical solution of the present invention, brief introduction will be made in conjunction with the accompanying drawings. Obviously, these drawings are merely some specific embodiments recorded in the present application. The technical solutions of the present invention include but not limited to these drawings.

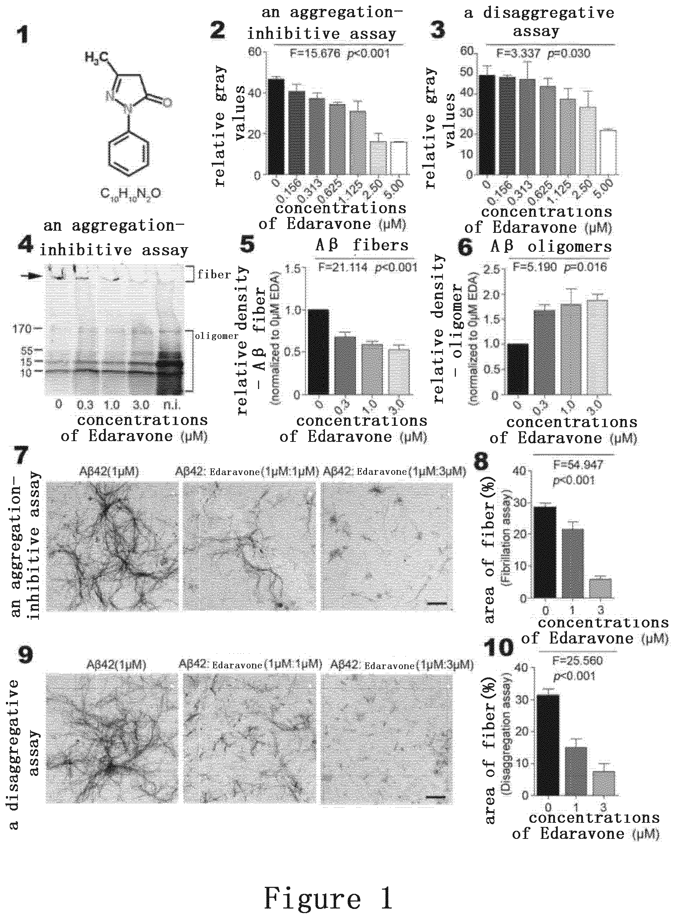

Figure 1 illustrates that Edaravone has a significant aggregation-inhibitive and disaggregative effect on Aβ. Wherein, 1: the molecular structure of Edaravone; 2 and 3: a Thioflavin T test demonstrates that Edaravone has a significant aggregation-inhibitive and disaggregative effecton on Aβ; 4-6: a Western blot further demonstrates that Edaravone has a significant aggregation-inhibitive effect on Aβ; 7-10: a transmission electron microscope further confirms that Edaravone has a significant aggregation-inhibitive and disaggregative effect on Aβ.

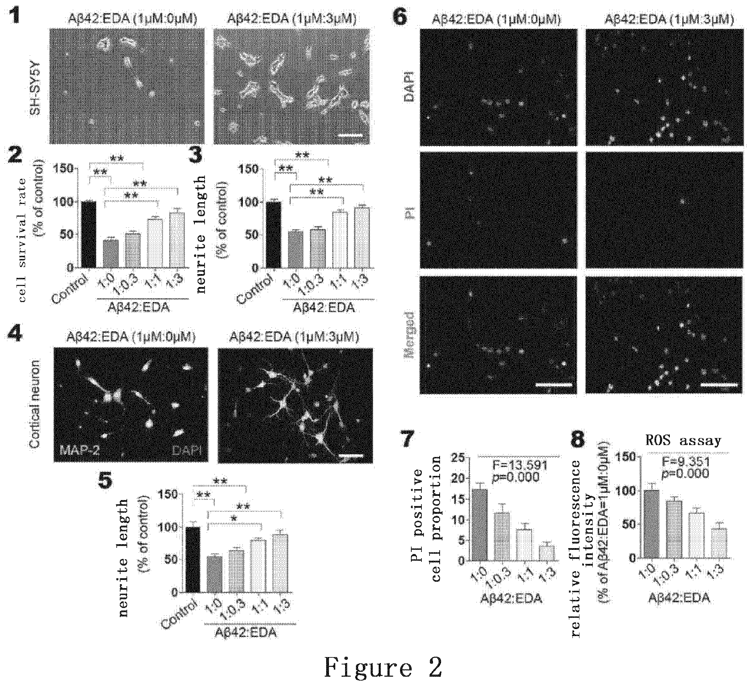

Figure 2 illustrates that Edaravone could antagonize the toxic effect of Aβ on cells. Wherein, 1-3: Edaravone could antagonize the toxic effect of Aβ on SH-SY5Y cells, wherein the cell survival rate and the cellular neurite length are both higher than the simple Aβ treated group; 4-5: Edaravone could antagonize the toxic effect of Aβ on primary cultured cortical neurons, wherein the neurite lengths of the cortical neuron in the Edaravone treated group are significantly higher than in the simple Aβ treated group; 6-7: Edaravone significantly reduces the Aβ induced apoptosis; 8: a ROS test demonstrates that Edaravone significantly reduces the intracellular oxidation level.

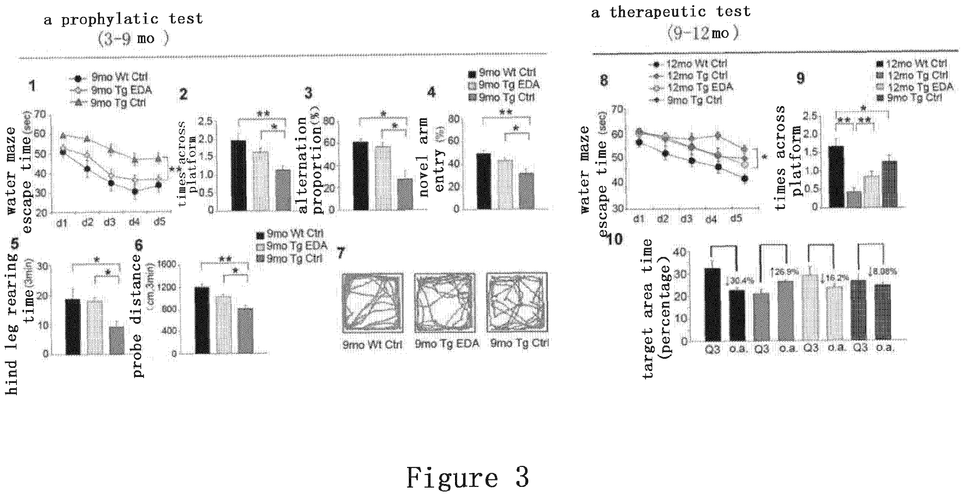

Figure 3 illustrates that Edaravone significantly improves the intelligent level of AD mice. Wherein, 1-2: a Morris water maze demonstrates that the escape latency and the times across the platform of the mice in the Edaravone prevention group are significantly lower than those in the control group; 3 and 4: the mice in the Edaravone prevention group perform much better in a Y-maze test than in the control group; 5-7: the mice in the Edaravone prevention group perform much better in an open field test than in the control group; 8-10: the mice in the Edaravone treatment group perform much better in the Morris water maze than in the control group.

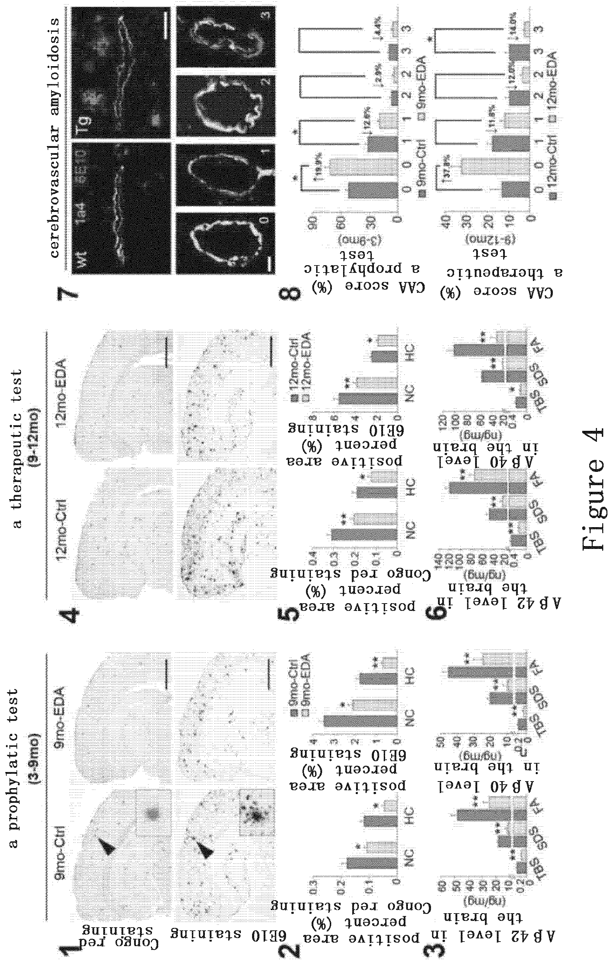

Figure 4 illustrates that Edaravone significantly reduces the Aβ deposition in the brain and in the blood vessels in the brain. Wherein 1-2: in the Edaravone prophylactic test, Edaravone significantly lowers the Aβ deposition in the brain; 4-5: in the Edaravone therapeutic test, Edaravone significantly lowers the Aβ deposition in the brain; 3 and 6: regardless in the prophylactic or therapeutic test, Edaravone could significantly reduce the Aβ level in the brain; 7-8: regardless in the prophylactic or therapeutic test, Edaravone could significantly reduce the Aβ deposition in the blood vessels in the brain.

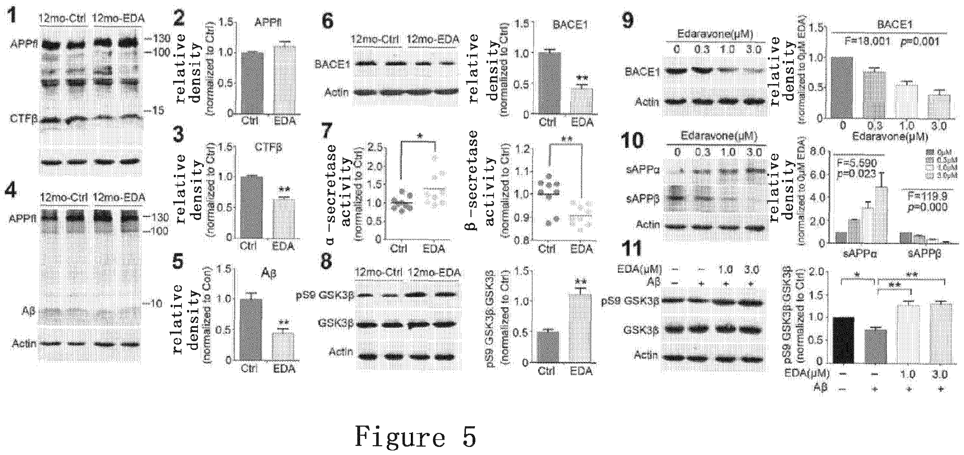

Figure 5 illustrates that Edaravone inhibits the BACE and Gsk-3β activities. Wherein: 1-5: Edaravone significantly reduces the CTF β and Aβ expressions in the brain of the AD mice; 6-7: Edaravone inhibits the BACE expressions and activities in the brain of the AD mice, and increases the α-secretase activities in the brain of the AD mice; 8: Edaravone inhibits the Gsk-3β activities in the brain of the AD mice; 9-10: an *in vitro* test further demonstrates that Edaravone inhibits the BACE expression; 11: *in vitro* test further demonstrates that Edaravone inhibits the Gsk-3β activities.

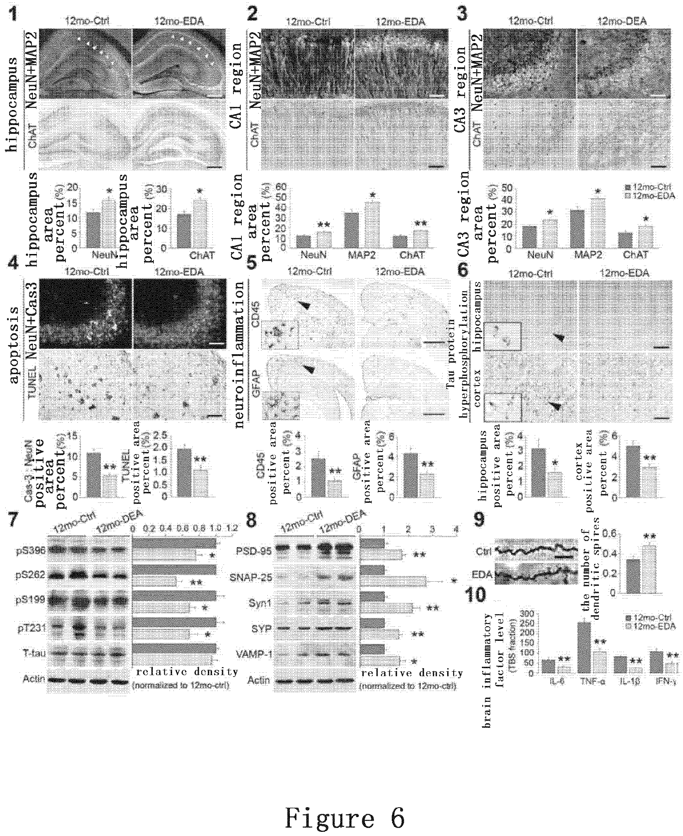

Figure 6 illustrates that Edaravone significantly improves the Aβ-induced secondary pathological changes. Wherein, 1-3: Edaravone could significantly reduce the damage to the structures of the neurons and the neuron losses in the brain of the AD mice; 4: Edaravone could significantly reduce the neuron apoptosis in the brain of the AD mice; 5: Edaravone could significantly reduce the reactivities of astrocytes and microglias in the brain of the AD mice; 6-7: Edaravone could significantly the phosphorylation degree of Tau proteins in the brain of the AD mice; 8-9: Edaravone could protect the integrity of the synapse structures in the brain of the AD mice; 10: Edaravone could significantly reduce the neuroinflammation in the brain of the AD mice.

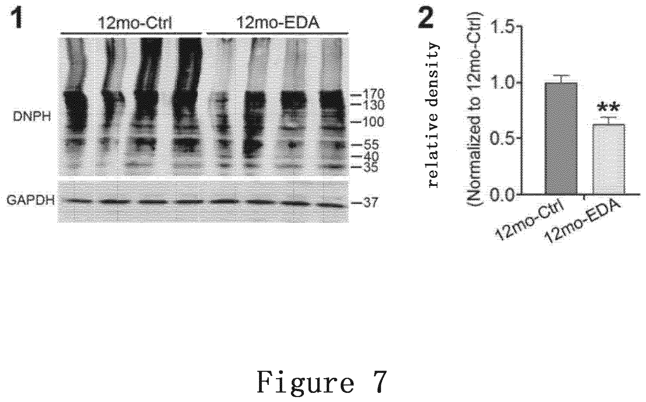

Figure 7 illustrates that Edaravone significantly reduces the oxidation level in the brain of the AD mice.

### DETAILED DESCRIPTION OF THE INVENTION

For further understanding the present invention, the preferred technical solutions of the present invention will be described in association with comperative examples below. These descriptions only illustrate the features and the advantages of the technical solutions of the present invention and do not limit the scope of the present invention.

### 1. A Thioflavin T fluorescence test

An inhibitive effect of Edaravone on Aβ aggregation: solutions of 10 µM Aβ 42 and different concentrations of Edaravone (0 µM, 1.56 µM, 3.13 µM, 6.25 µM, 11.25 µM, 25.0 µM, 50.0 µM and 100 µM) were incubated in an incubator at 37 °C for two days, a 5 µM thioflavin T working solution was added thereto and continued the incubation for 20 min, and optical density (OD) values were obtained under a microplate reader (excitation wavelength: 450 nm, emission wavelength: 482 nm).

A disaggregative effect of Edaravone on Aβ fibers: Aβ 42 was dissolved in the DMEM cultures and incubated at 37 °C for two days to form Aβ fibers. The solutions of the formed Aβ fibers and different concentrations of Edaravone (concrete concentrations are the same as above) were incubated under the same conditions for another three days, a 5 µM thioflavin T working solution was added and incubated for 20 min, OD values were obtained under the microplate reader (excitation wavelength and emission wavelength are the same as above).

### 2. A transmission electron microscope negative staining

A transmission electron microscope (TEM) negative staining was applied in the present research to further confirm the inhibitive effect on Aβ fiber formation and the disaggregative effect on the formed Aβ fibers of the Edaravone. Formvar coated copper grid was put on the bottom of a 24-well plate beforehand, and different concentrations of Edaravone working solutions were incubated together with Aβ in the 24-well plate. The treatment of Aβ and the subsequent incubation process are the same as in the thioflavin T test. After that, superfluous liquid was removed, stained using phosphotungstic acid for 30 s. The samples were naturally air-dried, observed and taken pictures by Joel 1200 EX transmission electron microscope.

### 3. A Western blot determination of the aggregation-inhibitive effect of Edaravone on Aβ

Solutions of 1 µM Aβ42 and different concentrations of Edaravone (0 µM, 0.3 µM, 1 µM and 3 µM) were incubated in a incubator at 37 °C for two days, the intermixture and a non-reductive loading buffer were mixed gently, Western blot was carried out with the detection antibody being a Aβ antibody (6E10) derived from a mouse .

### 4. A SH-SY5Y cell line culture, cell viability assay and neurite growth detection

The SH-SY5Y cell line was purchased from the Peking Union Hospital Cell Bank, cultured in a humidified incubator under 5% CO₂ and 37 °C. A MTT cell viability assay was performed according to the instructions in the kit (Sigma). The SH-SY5Y cells were treated with mixed working solutions of Aβ and different concentrations of Edaravone for 24 h, then were incubated with a MTT working solution (0.5 mg/ml) at 37 °C, then incubated with 10% SDS for 15 min. The optical density values were determined at a wavelength of 563 nm (Synergy H4, Bio Tek). The neurite growth test was performed according to the method described in the prior documents (see, for example, Wang, Y.J., et al., Effects of proNGF on neuronal viability, neurite growth and amyloid-beta metabolism. Neurotox Res, 2010, 17(3): p.257-67). The SH-SY5Y cells were cultured in the medium containing 1% FBS and 10 µM all-trans retinoic acid (RA) (Sigma, US) for 7 days, and the cells were treated with 1µM Aβ and different concentrations of Edaravone for 24 h, observed under the inverted microscope, taken pictures, and the neurite lengths of the 50 neurons per group were measured using ImageJ software.

### 5. A primary culture of cortical neurons, a ROS detection, and labeling apoptotic cells with propidium iodide (PI)

Cortical neurons were separated from the brains of the new born 129sv mice, and cultured on the poly-L-lysine coated cover glasses. 72 h later, the cells were treated with working solutions of 1 µM Aβ 42 oligomers and different concentrations of Edaravone for 24 h, then fixed with 4% paraform for 10 min, then stained with MAP-2 antibodies. The method for neurite length measurement is as mentioned above.

The ROS detection was performed according to the scheme provided by the manufacturer (Cell Biolabs Inc).

The method for labelling the apoptotic neurons with propidium iodide (PI) are as follows: living neurons were washed with a PBS solution for three times; the washed living neurons were incubated with a PI working solution (2 µg/ml) at room temperature for 15 min, and then washed with a HEPES buffer solution (100 mM HEPES, 140 mM NaCl, 25 mM CaCl2, pH 7.4), 5 min×3. The cells were finally fixed with 2% paraform in PBS for 20 min, then washed with buffer solution, 5 min×3, and incubated with DAPI (1:1000) 20 min for counterstaining. The cells were observed and taken pictures under B50fluorescence microscope.

### 6. Animals and Edaravone administration

APPswe/PS1DE9 (AD) transgenic mice were purchased from Jackson Laboratory, USA (No. 005864), raised in the SPF grade animal house of The Third Affiliated Hospital of The Third Military Medical University. All the processes and correlated experiments applied to the mice were approved and filed with the Animal Ethics Committee of the Third Military Medical University. In the prophylactic test, 3-month old AD mice received Edaravone treatment for six months, the blank control (AD mice) and normal wild type control (wild type litter mate) received isometrical normal saline. In the therapeutic test, 9-month old AD mice received Edaravone treatment for three months, blank control (AD mice) and normal wild type control at the same age were also set up. The equivalent therapeutic dose of Edaravone in mice was deduced from the dose for adults: Edaravone injection 12.6 mg/kg, ip, twice a week. Meanwhile, the mice of the other two groups were administered with Edaravone orally, with a daily dose of 0.1-25 mg/kg, for example, 3.5 mg/kg (both suitable for the prophylaxis and therapy purpose). Alternatively, the daily dose for the prophylactic group may be 6.4 mg/kg, and the daily dose for the treatment group may be 12.8 mg/kg. Administration from 3-month old to 9-month old is the prophylactic test, and administration from 9-month old to 12-month old is the therapeutic test. After reaching a stated time, the mice in each group received behavioral tests according to the method in the follows. Three mice in each group were taken for Golgi staining. The remaining mice were overly anaesthetized to receive brain anatomy. One side of the cerebral hemisphere was frozen and then for a biochemical analysis, and the other side of the cerebral hemisphere was fixed with 4% paraform for a histological analysis.

### 7. Behavioral tests

All the mice in each group received behavioral tests, including a water maze, Y-maze and open field test. Water maze test includes four days of platform test and the subsequent probe test. The entire process was recorded by video, and analyzed with an image analysis software (ANY-maze, Stoelting). In the platform test, the escape path length and escape latency were measured and calculated. In the probe test, the time in each quadrant and the time across platform were measured. The spontaneous alternation test and novel arm probe test were carried out in the Y-maze. In the spontaneous alternation test, the mouse was free running in the maze for 5 min. Alternation is defined as entering the three arms connected with each other continuously. The percentage of alternation is the ratio of actual number of alternation to the maximum number of possible alternation (defined as the arm entry minus 2) multiplied by 100. In the novel arm probe test, one arm (novel arm) is blocked, the mouse was allowed to probe in the other two arms for 5 min. After a two-hour intermission, the mouse may probe the three arms freely. In the open field test, the mouse was put in the center of an open field for 3 min, and its path was recorded by a tracking system (Limelight, ActiMetrics). At the same time, the numbers of rearing, grooming, defecation and urination of the mouse were recorded.

### 8. A quantitative analysis of the Aβ deposition in the brain

The compact Aβ plaque was stained and labelled with Congo red. A equidistant (∼ 1.3 mm) frozen section was treated with a sodium chloride working solution at room temperature for 20 min, with a Congo red working solution treated for 45 min, and rapidly dehydrated with absolute alcohol. The total Aβ plaques were labelled with 6E10 immunohistochemistry. 6E10 staining was performed by a method of bleach section. The Aβ plaques in the neopallium and hippocampal area were quantitatively analyzed with ImageJ analysis software to obtain the area percentage of the Aβ plaque and the quantities in a unit area.

### 9. A cerebrovascular amyloidosis detection

A new assessment method was applied in the present research to assess the severity of the cerebrovascular amyloidosis. The blood vessels in the brain were double immunofluorescence stained with α-smooth muscle action antibody (1a4) and Aβ antibody (6E10), observed under confocal laser microscopy (Radiance 2000MP, Bio-Rad), and the images of the blood vessels were recorded. The severity of the CAA of each blood vessel was scored based on the following four-level scale: level 0: no Aβ deposition; level 1: little Aβ deposition, Aβ deposition occupies less than one third of the perimeter of the blood vessel; level 2: moderate Aβ deposition, Aβ deposition occupies more than one third but less than two thirds of the perimeter of the blood vessel; level 3: severe deposition, Aβ deposition occupies more than 75% of the perimeter of the blood vessel.

### 10. Staining and quantitative analysis of microbleeds

First, 2% potassium ferrocyanide was used to stain the hemosiderin, then 1% Nuclear Fast Red was used to counterstain the cell nucleus. The microbleeds in each brain tissue slice were quantified under microscopy, and the average of the hemosiderin precipitations contained in each slice was calculated.

### 11. ELISA analysis

After homogenating the frozon brain tissues, extraction was conducted with TBS, SDS and formic acid successively. The TBS, SDS and formic acid extracts represent soluble Aβ, loosen senile plaques and compact senile plaques, respectively. An ELISA kit (Covance or eBioscience) was used to determine the concentrations of Aβ 40, Aβ 42 (Covance), IL-6, IL-1β, INF-γ, TNF-β (eBioscience, BMS603, BMS6002, BMS606, BMS607) in the brain and serum. The determination steps were carried out according to the instructions of the kit. The determinations of the activities of alpha and beta secretases in the brain tissues were also carried out according to the instructions of the kit (R&D Systems).

### 12. Immunostaining of neuron NeuN, ChAT and MAP-2

To clarify the protective effect of Edaravone on neurons, immunohistochemistry detections of NeuN, ChAT and MAP-2 in the method of bleach section were carried out to the brain tissue slices. Primary antibodies were rabbit-anti-mouse NeuN, ChAT and MAP-2 antibodies, respectively, with a dilution ratio of 1:200, incubated at 4 °C overnight, biotinylated Ig secondary antibodies were incubated at 37 °C for 2 h, followed by DAB colouration, serial dehydration, transparentized and mounting.

### 13. Astrocyte reaction, microglia reaction and Tau hyperphosphorylation immunostaining

Immunohistochemistry in the method of bleach section was carried out according to the previous steps. The primary antibodies were rabbit-anti-mouse CD45 antibody (for labelling microglias), rabbit-anti-mouse GFAP (for labelling astrocytes) and rabbit-anti-mouse pSer396-Tau antibody, respectively. The dilution ratio was 1:200, the following steps were the same as above.

### 14. Golgi staining

After overly anesthetized, the animals received heart perfusions with normal saline containing 0.5% nitrous acid, 4% paraform, and Golgi dye liquor, successively. After perfusion, the brain tissues were cut into 0.1cm × 0.1cm pieces, transferred to brown vessels containing Golgi dye liquor, left without light at room temperature for 3 d, and then immersed in the 1% silver nitrate solution for further incubation, reacted without light for 3 d. The brain tissues were sliced with vibratome (Leica, VT1000 S, Germany) at a slice thickness of 35 um. The numbers of the dendritic spines within 10 µm of the central dendrites of the pyramidal cells in the hippocampus CA1 region were accounted.

### 15. Determination of an oxidation level in the brain tissues with dinitrophenylhydrazine (DNPH) Western blotting

The carbonyl level of the proteins in the tissue reflects the oxidation level in the tissue. DNPH is reacted with the carbonyl of the proteins to form a protein-DNP hydrazone complex, and the level of the complex can be detected with DNPH antibodies so as to assess the oxidation level in the tissue. A 5 µl sample of fresh brain tissues was co-incubated with 12% SDS and a 10 µl DNPH working solution at room temperature for 20 min. The sample was neutralized with a 7.5 µl neutralization solution. Samples containing 15 µg total proteins were loaded and detected with Western blot. Overoxidation or oxidation overbalance is an important aspect of the AD pathological mechanism, which facilitates Aβ deposition and formation of neurofibrillary tangles (see, for example, Oxidative Stress and its Implications for Future Treatments and Management of Alzheimer Disease, Int J Biomed Sci, Clark, T.A., et al., 2010, 6(3): p.225-227).

### 16. Western blot

The enzymes related with Aβ formation and degradation their molecule levels, the levels of hyperphosphorylated Tau proteins, and the levels of synaptic plasticity correlated proteins were all detected with Western blot. The proteins in the brain tissue homogenates were extracted with a RIPA buffer solution. The samples were transferred to a SDS-PAGE (4-10% acrylamide) gel. The separated proteins were transferred to a nitrocellulose membrane. The blotting was detected with the following antibodies: APP C terminal antibody (171610, Millipore), BACE1 antibody (Millipore), Aβ antibody (6E10, Abcam), Neprilysin antibody (Millipore), RAGE antibody (Millipore), LRP antibody (5A6, Calbiochem), IDE antibody (epitomics), phosphorylated Tau protein antibody, including pS396 (Signalway), pT231 (Signalway), pS199 (epitomics), pS262 (Abcam), SNAP25 antibody (Millipore), Synaptophysin antibody (Millipore), Synapsin I antibody (Millipore), VAMP1 antibody (Millipore), PSD95 antibody (Millipore), F-actin antibody (Actin) (Sigma-Aldrich). After incubated with IRDye 800CW secondary antibodies, an Odyssey fluorescence scanner is used for scanning.

The results of the present application show that Edaravone has significant intervention effects on multiple key aspects of the Aβ pathologic process:

### (1) Edaravone inhibits Aβ from aggregating into fibers, and facilitates the Aβ fiber disaggregation

The thioflavin T test demonstrates that Edaravone could effectively inhibit Aβ aggregation in a dose-dependent manner, the higher the concentration of Edaravone, the more significant the effect. At the same time, the disaggregation test demonstrates that Edaravone could also disaggregate the formed Aβ fibers or larger aggregates effectively, the higher the concentration of Edaravone, the more significant this effect (see Figure 1: 2 and 3). The Western blot further verifies the above conclusion. It is found that 3.0 µM Edaravone could inhibit 50% Aβ from forming aggregates (see Figure 1: 4-6). The TEM test shows that 3.0 µM Edaravone significantly inhibits Aβ aggregation, Aβ may even unobservable in part of the copper grid. At the same time, the TEM finds that 3.0 µM Edaravone could disaggregate nearly 80% Aβ fibers (see Figure 1: 7-10).

### (2) Edaravone antagonizes the cytotoxicity effect of Aβ

To explore whether Edaravone has an effect of antagonizing Aβ cytotoxicity, SH-SY5Y cell lines and primary cultured cortical neurons were used as the object in the present research, which were added 1 µM Aβ oligomers and treated with different concentrations of Edaravone, and the cell survival rate and the neurite growth were observed. First, it is found in the SH-SY5Y test that 1 µM Aβ oligomers will lead to a substantial cell death (living cells are about 40% of the normal control group) and extensive neurite collapse (neurite length is about 50% of the normal control group). After adding Edaravone, cell survival rate increases significantly, and the cell survival rate increases with the rise of the concentration of Edaravone in a significant dose-dependent manner. At the same time, after adding Edaravone, all-trans-Retinoic acid induced neurite length also increases (see Figure 2: 1-3). In the primary cultured cortical neuron test, the above effect is also observed (see Figure 2: 4 and 5). Second, it is found in the ROS test that Edaravone could reverse the Aβ induced oxidative stress (see Figure 2: 8). In the PI test, Edaravone treatment group has an apoptotic cell ratio significantly lower than in the simple Aβ control group (see Figure 2: 6 and 7).

### (3) The application of Edaravone significantly improves the behavioral performance of the AD mice

Based on the Morris water maze test, it is found by the present inventors that, no matter in the prophylactic test (see Figure 3: 1-2) or in the therapeutic test (see Figure 3: 8-10), behavioral performance of the AD mice in Edaravone treatment group is significantly better than in the control group, presented as the reduction of escape time and the increase in times across platform. It is demonstrated by the Morris water maze that Edaravone could improve the learning and space memory abilities of the AD mice. To comprehensively assess the improvement of the Edaravone on the intelligence of the AD mice, Y-maze, open field test, etc. were also carried out in the present research successively. It is found that, in the spontaneous alternation test and novel arm probe test, the arm entry of the AD mice in the Edaravone treatment group is significantly higher than in the control group, the proportion of the novel arm entry is significantly increased, the alternation proportion is also significantly increased, suggesting that Edaravone could improve the exploring ability and working memory capacity of the AD mice (see Figure 3: 3 and 4). It is found by the inventors in the open field test that the rearings and the probe distance of the Edaravone treated mice are significantly higher than in the control group (see Figure 3: 5-7). Similar results are obtained both through ip administration and p.o. administration. It is demonstrated that Edaravone treatments through different routes of administration could improve the intelligence level of the AD mice.

### (4) Edaravone significantly reduces the deposition of Aβ in the blood vessels of the brain

It is found by Congo red staining and 6E10 immunostaining that Edaravone could significantly reduce the Aβ deposition in the brain, being about 50% of the control group (see Figure 4: 1-2 and 4-5). The most valuable is that it is found by double immunofluorescence staining the vascular wall and Aβ, Aβ deposition in the blood vessels of the brain in the Edaravone treatment group is significantly reduced, the proportions of the blood vessels at level 0 and level 1 CAA are significantly increased, the proportions of the blood vessels at level 2 and level 3 are significantly reduced (see Figure 4: 7 and 8). At the same time, the brain tissues of the AD mice were extracted with TBS, 2% SDS and FA, and extracts of different Aβ aggregation states were obtained, respectively, and the Aβ 40, Aβ 42 levels in the extracts were detected using Covance ELISA kit, It is found that Edaravone could significantly reduce the levels of Aβ at different aggregation states (see Figure 4: 3 and 6), which is consistent with the *in vitro* experimental result. An oral administration also receives the similar result as above.

### (5) Edaravone reduces the formation of Aβ by inhibiting BACE1 expression

It is found by Western blot that Edaravone could reduce the formation of Aβ in the brain, decrease the formation of CTFβ (see Figure 5: 1-5). To explore the mechanism, it is found that Edaravone has no influence on Aβ metabolizing enzymes such as NEP, IDE, RAGE, LRP, etc., but has a significant influence on α-secretase and β-secretase (BACE1), the cleavage enzymes of APP, presented as the increase of the activity of α-secretase and the significant decrease of the expression and the activity of β-secretase in the Edaravone treatment group (see Figure 5: 6-7 and 9-10). At the same time, it is also found that Edaravone could inhibit the activity of Gsk-3β (see Figure 5: 8 and 11).

### (6) Edaravone could reduce the microglia, astrocyte and neural inflammatory reactions

The microglia, astrocyte and neural inflammatory reactions in the brains of AD mice in both prophylactic test and therapeutic test are all reduced significantly. The microglia, astrocyte and neural inflammatory reaction levels in the brains of AD mice in the Edaravone treatment group (p.o.) are significantly reduced, too (see Figure 6: 5 and 10).

### (7) Edaravone reduces the death of neurons and synaptic degeneration

The losses of neurons and the synaptic degeneration are typical clinical manifestations of AD. To explore whether Edaravone has neuron protective effect, immunohistochemistry for MAP-2, NeuN, ChaT etc. was carried out by the inventors. It is found that, as compared with the control group, the NeuN and ChaT positive area ratios are significantly increased in the Edaravone treatment group. It is found by the Golgi staining that the numbers of the dendritic spines are also significantly increased. It is found by Western blot that the expressions of the synapse associated proteins in the Edaravone treatment group are much higher than in the control group (see Figure 6: 1-3, 4 and 8-9).

### (8) Edaravone reduces the phosphorylation level of Tau protein

It is found in Western blot that Edaravone could reduce the phosphorylation level of Tau protein at multiple pathological sites (see Figure 6: 6 and 7). At the same time, it is found in the p396-Tau immunohistochemistry that the p396-Tau positive area ratio is much lower in the Edaravone treatment group.

The descriptions of the above specific embodiments are only for helping understanding the core concept of the present invention. It should be noted that, various improvements and modifications may be made to the technical solutions of the present invention without departing from the principles of the present invention to those skilled in the art, although these improvements and modifications also fall into the range of the claims in the present invention.

## Claims

1. Edaravone, an Edaravone analogue, wherein (1) the methyl at position 3 of the pyrazoline ring is replaced with a lower (C₁₋₆) alkyl other than methyl, or with a lower alkoxy, (2) the methyl at position 3 of the pyrazoline ring is replaced with hydrogen while the hydrogen at position 4 is substituted with a lower alkyl or alkoxy, and/or (3) phenyl is optionally substituted with one or more substituents selected from a lower alkyl, a lower alkoxy, nitro, and halogen, or an Edaravone derivative, wherein the ketone in position 5 of the pyrazoline ring is transformed into enol that can react with a carboxylic acid to generate an ester, which in turn can be converted into a ketone after hydrolysis in vivo, for use in the treatment of cerebral amyloid angiopathy (CAA) in a patient.

2. Edaravone, the Edaravone analogue or derivative for use according to claim 1, wherein Edaravone, the Edaravone analogue or derivative is administered intravenously, subcutaneously or orally.

3. Edaravone, the Edaravone analogue or derivative for use according to claim 1, wherein Edaravone, the Edaravone analogue or derivative is formulated into an injection, a tablet or a capsule.

4. Edaravone, the Edaravone analogue or derivative for use according to claim 1, wherein Edaravone, the Edaravone analogue or derivative is administered in combination with other CAA therapeutic medicine.

5. Edaravone, the Edaravone analogue or derivative for use according to claim 4, wherein said other medicine is an Aβ antibody.

6. Edaravone, the Edaravone analogue or derivative for use according to claim 1, further comprising determining that the patient suffers from CAA before administration.

7. Edaravone, the Edaravone analogue or derivative for use according to claim 6, wherein the determining refers to determining clinical symptoms of the patient suffering from CAA.

8. Edaravone, the Edaravone analogue or derivative for use according to claim 1, wherein the patient has or does not have characteristic plaques of Alzheimer's disease in the brain.

9. Edaravone, the Edaravone analogue or derivative for use according to claim 1, wherein a daily dose of Edaravone, the Edaravone analogue or derivative is between 0.1mg/kg and 25 mg/kg.

10. Edaravone, an Edaravone analogue wherein (1) the methyl at position 3 of the pyrazoline ring is replaced with a lower (C₁₋₆) alkyl other than methyl, or with a lower alkoxy, (2) the methyl at position 3 of the pyrazoline ring is replaced with hydrogen while the hydrogen at position 4 is substituted with a lower alkyl or alkoxy, and/or (3) phenyl is optionally substituted with one or more substituents selected from a lower alkyl, a lower alkoxy, nitro, and halogen, or an Edaravone derivative wherein the ketone in position 5 of the pyrazoline ring is transformed into enol that can react with a carboxylic acid to generate an ester, which in turn can be converted into a ketone after hydrolysis in vivo, for use in the prevention of CAA in a patient susceptible to CAA.

## Patentansprüche

1. Edaravon, ein Edaravon-Analogon, wobei (1) das Methyl in Position 3 des Pyrazolinrings durch ein Niederalkyl (C₁₋₆), das nicht Methyl ist, oder durch ein Niederalkoxy ersetzt ist, (2) das Methyl in Position 3 des Pyrazolinrings durch ein Wasserstoffatom ersetzt ist, während das Wasserstoffatom in Position 4 durch ein Niederalkyl oder Niederalkoxy substituiert ist, und/oder (3) das Phenyl wahlweise durch einen oder mehrere Substituenten, der/die aus einem Niederalkyl, einem Niederalkoxy, einer Nitrogruppe, einem Halogen oder einem Edaravon-Derivat ausgewählt ist/sind, substituiert ist, wobei das Keton in Position 5 des Pyrazolinrings in ein Enol umgewandelt wird, das mit einer Carbonsäure umgesetzt werden kann, um einen Ester zu bilden, der nach der Hydrolyse in vivo wiederum in ein Keton umgewandelt werden kann, zur Verwendung bei der Behandlung von zerebraler Amyloidangiopathie (CAA) bei einem Patienten.

2. Edaravon, das Edaravon-Analogon oder -Derivat zur Verwendung nach Anspruch 1, wobei Edaravon, das Edaravon-Analogon oder -Derivat intravenös, subkutan oder oral verabreicht wird.

3. Edaravon, das Edaravon-Analogon oder -Derivat zur Verwendung nach Anspruch 1, wobei Edaravon, das Edaravon-Analogon oder -Derivat als Injektion, Tablette oder Kapsel formuliert ist

4. Edaravon, das Edaravon-Analogon oder -Derivat zur Verwendung nach Anspruch 1, wobei Edaravon, das Edaravon-Analogon oder -Derivat in Kombination mit anderen therapeutischen Arzneimitteln für CAA verabreicht wird..

5. Edaravon, das Edaravon-Analogon oder -Derivat nach Anspruch 4, wobei das andere Arzneimittel ein Aß-Antikörper ist.

6. Edaravon, das Edaravon-Analogon oder -Derivat zur Verwendung nach Anspruch 1, ferner umfassend das Bestimmen, dass der Patient an CAA leidet, vor der Verabreichung.

7. Edaravon, das Edaravon-Analogon oder -Derivat zur Verwendung nach Anspruch 6, wobei das Bestimmen das Bestimmen der klinischen Symptome des Patienten, der an CAA leidet, betrifft.

8. Edaravon, das Edaravon-Analogon oder -Derivat zur Verwendung nach Anspruch 1, wobei der Patient für Morbus Alzheimer charakteristische Plaque im Gehirn aufweist oder nicht.

9. Edaravon, das Edaravon-Analogon oder -Derivat zur Verwendung nach Anspruch 1, wobei eine Tagesdosis von Edaravon, dem Edaravon-Analogon oder -Derivat von 0,1 mg/kg bis 25 mg/kg beträgt.

10. Edaravon, ein Edaravon-Analogon, wobei (1) das Methyl in Position 3 des Pyrazolinrings durch ein Niederalkyl (C₁₋₆), das nicht Methyl ist, oder durch ein Niederalkoxy ersetzt ist, (2) das Methyl in Position 3 des Pyrazolinrings durch ein Wasserstoffatom ersetzt ist, während das Wasserstoffatom in Position 4 durch ein Niederalkyl oder Niederalkoxy substituiert ist, und/oder (3) das Phenyl wahlweise durch einen oder mehrere Substituenten, der/die aus einem Niederalkyl, einem Niederalkoxy, einer Nitrogruppe, einem Halogen oder einem Edaravon-Derivat ausgewählt ist/sind, substituiert ist, wobei das Keton in Position 5 des Pyrazolinrings in ein Enol umgewandelt wird, das mit einer Carbonsäure umgesetzt werden kann, um einen Ester zu bilden, der nach der Hydrolyse in vivo wiederum in ein Keton umgewandelt werden kann, zur Verwendung bei der Prävention von CAA bei einem Patienten, der anfällig für CAA ist.

## Revendications

1. Edaravone, un Edaravone analogue, où (1) le méthyle à la position 3 de l'anneau pyrazoline est remplacé avec un (C₁₋₆) alkyle inférieur autre que le méthyle, ou avec un alkoxy inférieur, (2) le méthyle à la position 3 de l'anneau pyrazoline est remplacé par de l'hydrogène pendant que l'hydrogène à la position 4 est substitué avec un alkyle inférieur ou alkoxy, et/ou (3) le phényle est éventuellement substitué avec un ou plusieurs substituants choisis d'entre un alkyle inférieur, un alkoxy inférieur, un nitro, et un halogène, ou un Edaravone dérivative, où la cétone dans la position 5 de l'anneau pyrazoline est transformé en énol qui peut réagir avec un acide carboxylique pour la génération d'un ester, qui à son tour peut être converti dans une cétone après l'hydrolyse in vivo, pour utilisation dans le traitement de l'angiopathie amyloïde cérébrale (CAA) chez un patient.

2. Edaravone, l'Edaravone analogue ou dérivative pour utilisation selon la revendication 1, où l'Edaravone, l'Edaravone analogue ou dérivative est administré par voie intraveineuse, sous-cutanée ou orale.

3. Edaravone, l'Edaravone analogue or dérivative pour utilisation selon la revendication 1, où l'Edaravone, l'Edaravone analogue ou dérivative est formuléen une injection, un comprimé ou une capsule.

4. Edaravone, l'Edaravone analogue ou dérivative pour utilisation selon la revendication 1, où l'Edaravone, l'Edaravone analogue ou dérivative est administré en combinaison avec d'autres médicaments thérapeutiques CAA.

5. Edaravone, l'Edaravone analogue ou dérivative pour utilisation selon la revendication 4, où ledit autre médicament est un anticorps Aβ.

6. Edaravone, l'Edaravone analogue ou dérivative pour utilisation selon la revendication 1, comprenant en outre la détermination du fait que le patient souffrede CAA avant l'administration.

7. Edaravone, l'Edaravone analogue ou dérivative pour utilisation selon la revendication 6, où la détermination se réfère à la détermination des symptômes cliniques du patient souffrant de CAA.

8. Edaravone, l'Edaravone analogue ou dérivative pour utilisation selon la revendication 1, où le patient a ou n'a pas de plaques caractéristiques de la maladie d'Alzheimer dans le cerveau.

9. Edaravone, l'Edaravone analogue ou dérivative pour utilisation selon la revendication 1, où une dose quotidienne d'Edaravone, l'Edaravone analogue ou dérivative est entre 0,1 mg/kg et 25 mg/kg.

10. Edaravone, un Edaravone analogue où (1) le méthyle à la position 3 de l'anneau pyrazoline est remplacé avec un (C₁₋₆) alkyle inférieur autre que le méthyle, ou avec un alkoxy inférieur, (2) le méthyle à la position 3 de l'anneau pyrazoline est remplacé par de l'hydrogène pendant que l'hydrogène à la position 4 est substitué avec un alkyle inférieur ou alkoxy, et/ou (3) le phényle est éventuellement substitué avec un ou plusieurs substituants choisis d'entre un alkyle inférieur, un alkoxy inférieur, un nitro, et un halogène, ou un Edaravone dérivative, où la cétone dans la position 5 de l'anneau pyrazoline est transformé en énol qui peut réagir avec un acide carboxylique pour la génération d'un ester, qui à son tour peut être converti dans une cétone après l'hydrolyse in vivo, pour utilisation dans la prévention de CAA chez un patient susceptible de CAA.
